(19) Europäisches Patentamt
European Patent Office
Office européen des brevets

(11) **EP 3 817 800 B1**

(12) **EUROPÄISCHE PATENTSCHRIFT**

(45) Veröffentlichungstag und Bekanntmachung des Hinweises auf die Patenterteilung:
**06.09.2023   Patentblatt 2023/36**

(21) Anmeldenummer: **19748647.5**

(22) Anmeldetag: **05.07.2019**

(51) Internationale Patentklassifikation (IPC):
*A61M 16/20* (2006.01)    *A61M 16/00* (2006.01)

(52) Gemeinsame Patentklassifikation (CPC):
**A61M 16/209; A61M 16/205; A61M 16/206;**
A61M 16/00; A61M 16/0066; A61M 16/022;
A61M 2016/0027; A61M 2016/0033;
A61M 2016/0039; A61M 2016/0042; A61M 2205/16;
A61M 2205/3334; A61M 2205/3368

(86) Internationale Anmeldenummer:
**PCT/DE2019/000186**

(87) Internationale Veröffentlichungsnummer:
**WO 2020/007389 (09.01.2020 Gazette 2020/02)**

(54) **GASSTEUERUNGSVORRICHTUNG FÜR EIN BEATMUNGSGERÄT**

GAS CONTROL DEVICE FOR A VENTILATOR

DISPOSITIF DE COMMANDE DE GAZ POUR UN APPAREIL RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.07.2018   DE 102018005341**

(43) Veröffentlichungstag der Anmeldung:
**12.05.2021   Patentblatt 2021/19**

(73) Patentinhaber: **Löwenstein Medical Technology S.A.**
**2557 Luxembourg (LU)**

(72) Erfinder:
• **GARDEIN, Joachim**
**38430 Icod de los Vinos,  Teneriffa (ES)**
• **HAHN, Henry**
**22525 Hamburg (DE)**
• **ADAMETZ, Benjamin**
**22609 Hamburg (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
EP-A1- 3 536 369         WO-A1-2017/059667
CN-A- 107 308 531        DE-U1-202011 102 764
DE-U1-202017 005 964

EP 3 817 800 B1

**Beschreibung**

Die vorliegende Erfindung betrifft eine Gassteuerungsvorrichtung für ein Beatmungsgerät umfassend einen ersten Gaskanal mit einem Rückschlagventil, einen zweiten Gaskanal und eine Schalteinrichtung. Die Gassteuerungsvorrichtung kann in dem Beatmungsgerät angeordnet sein oder dem Beatmungsgerät zugeordnet sein.

Beatmungsgeräte werden für die Therapie respiratorischer Störungen eingesetzt, dabei können die Beatmungsgeräte in der nicht-invasiven und invasiven Beatmung, sowohl innerklinisch als auch außerklinisch, verwendet werden.

Bei einer Beatmung eines Patienten kann in der Regel ein Beatmungsgerät mit einem inspiratorischen Zweig für den Atemgasfluss und optional mit einem Zweig für den exspiratorischen Atemgasfluss eingesetzt werden. Der Zweig für den exspiratorischen Atemgasfluss ermöglicht die Ausatmung/Exspiration eines Atemgases durch den Patienten, während der Zweig für den inspiratorischen Atemgasfluss den Patienten mit Atemgas versorgt.

Bei den im Stand der Technik bekannten Beatmungsgeräten kann es zu Blockaden/Störungen im exspiratorischen Zweig des Beatmungsgerätes kommen.

Wenn der inspiratorische Zweig des Beatmungsgerätes ein Rückschlagventil umfasst, ist eine Rückführung ausgeatmeten Atemgases in das Beatmungsgerät nicht möglich. Der Patient ist dann an einer Ausatmung gehindert.

WO 2017/059667 und DE 20 2017 005964 offenbaren Gassteuerungsvorrichtungen für Beatmungsgeräte.

Es ist daher Aufgabe der vorliegenden Erfindung, eine Vorrichtung zur Verfügung zu stellen, die eine Exspiration von Atemgas auch bei einer Störung/Blockade eines, insbesondere exspiratorischen, Zweiges eines Beatmungsgerätes sicherstellt.

Es ist auch Aufgabe der vorliegenden Erfindung, eine Vorrichtung zur Verfügung zu stellen, die einen bedarfsweise aktivierbaren Gaskanal bereitstellt.

Diese Aufgabe wird durch eine Gassteuerungsvorrichtung gemäß dem Anspruch 1 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die vorliegende Erfindung betrifft eine Gassteuerungsvorrichtung für ein Beatmungsgerät umfassend einen ersten Gaskanal mit einem ersten Ventil, wobei der erste Gaskanal und das Ventil eingerichtet sind, einen unter Druck stehenden Gasstrom in einer ersten Richtung passieren zu lassen und einen unter Druck stehenden Gasstrom in einer zweiten Richtung zu sperren, wobei eine Öffnung von dem ersten Gaskanal abzweigt und zu einem Umgehungskanal, für das Ventil, führt, wobei ein zweites Ventil eingerichtet ist, einen Gasstrom vom dem ersten Gaskanal zu dem Umgehungskanal zu sperren und/oder zumindest zeitweise zu ermöglichen, wobei eine Schalteinrichtung eingerichtet ist das zweite Ventil zu steuern, einen Gasstrom vom dem ersten Gaskanal zu dem Umgehungskanal zu sperren und/oder zumindest zeitweise zu ermöglichen.

Die vorliegende Erfindung betrifft auch eine Gassteuerungsvorrichtung umfassend einen ersten Gaskanal mit einem ersten Ventil, wobei der erste Gaskanal und das Ventil eingerichtet sind, einen unter Druck stehenden Gasstrom in einer ersten Richtung passieren zu lassen und einen unter Druck stehenden Gasstrom in einer zweiten Richtung zu sperren, wobei eine Öffnung von dem ersten Gaskanal abzweigt und zu einem Umgehungskanal, für das Ventil, führt, wobei ein zweites Ventil eingerichtet ist, einen Gasstrom vom dem ersten Gaskanal zu dem Umgehungskanal zu sperren und/oder zumindest zeitweise zu ermöglichen, wobei eine Schalteinrichtung eingerichtet ist das zweite Ventil zu steuern, einen Gasstrom vom dem ersten Gaskanal zu dem Umgehungskanal zu sperren und/oder zumindest zeitweise zu ermöglichen.

Die vorliegende Erfindung ist auch dadurch gekennzeichnet, dass die zweite Richtung entgegengesetzt zur ersten Richtung ist.

Die vorliegende Erfindung ist auch dadurch gekennzeichnet, dass das erste Ventil ein pneumatisch betätigtes Rückschlagventil ist, welches durch die Kraft des unter Druck stehenden Gasstroms in der zweiten Richtung betätigt wird und somit der Gasstrom das Rückschlagventil in einer zweiten Richtung nicht passieren kann.

Die vorliegende Erfindung ist auch dadurch gekennzeichnet, dass der Gasstrom der zweiten Richtung, Atemgas der Exspiration ist und der Gasstrom der ersten Richtung Atemgas der Inspiration ist.

Die vorliegende Erfindung ist auch dadurch gekennzeichnet, dass eine Atemgasquelle Atemgas aus der Umgebung ansaugt und entlang dem ersten Gaskanal für die Inspiration in der ersten Richtung zu dem Schlauchstutzen und dem Beatmungsschlauch befördert, wobei der Gasstrom das geöffnete Rückschlagventil passiert.

Die vorliegende Erfindung ist auch dadurch gekennzeichnet, dass die Schalteinrichtung das Ventil öffnet und so der unter Druck stehende Gasstrom der Exspiration durch die Öffnung und durch den Umgehungskanal zumindest teilweise in die Umgebung gelangt.

Die vorliegende Erfindung ist auch dadurch gekennzeichnet, dass das Ventil als pneumatisches Ventil ausgeführt ist, wobei eine Dichtung des Ventils die gasleitende Verbindung zwischen Öffnung und Umgebungskanal verschließt oder öffnet und wobei ein zweiter Gaskanal eingerichtet ist einen pneumatischen Steuerdruck auf die Oberfläche der Dichtung zu leiten.

Die vorliegende Erfindung ist auch dadurch gekennzeichnet, dass die mit dem Steuerdruck beaufschlagte Dichtung eingerichtet ist, sich dichtend an den Rand der Öffnung anzulegen und so einen Gasstrom von der Öffnung in den Umgehungskanal zu verhindern.

Die vorliegende Erfindung ist auch dadurch gekennzeichnet, dass der zweite Gaskanal mit dem Beatmungsschlauch oder dem Gaskanal pneumatisch verbunden ist und die Schalteinrichtung die pneumatische Verbindung zumindest zeitweise öffnet oder verschließt.

Die vorliegende Erfindung betrifft auch eine Gassteuerungsvorrichtung für ein Beatmungsgerät umfassend einen ersten Gaskanal mit einem Rückschlagventil, einen zweiten Gaskanal und eine Schalteinrichtung, wobei der erste Gaskanal eine Öffnung zum Ausströmen von Gas mit einer Dichtung und einer Verschlusskappe aufweist.

Erfindungsgemäß ist die Gassteuerungsvorrichtung eingerichtet, bei einer Blockade/Störung eines exspiratorischen Zweiges des Beatmungsgerätes den Gasstrom des ersten Gaskanals an dem Rückschlagventil vorbeizuführen. Dadurch wird bei einer Blockade/Störung des exspiratorischen Zweiges eine Ausatmung des Patienten sichergestellt. Beispielsweise ist der erste Gaskanal mit dem zweiten Gaskanal verbunden. Eine getrennte Ausbildung des ersten Gaskanals und des zweiten Gaskanals, wobei der zweite Gaskanal durch einen separaten Gaskanal gespeist wird, ist in einer alternativen Ausgestaltung denkbar. Auch ist es denkbar, die erfindungsgemäße Gassteuerungsvorrichtung ebenfalls bei einer Blockade/Störung in einem inspiratorischen Zweig des Beatmungsgerätes einzusetzen. In einem solchen Fall könnte die Gassteuerungsvorrichtung eingesetzt werden, um eine Inspiration eines Patienten bei einer Blockade/Störung in dem inspiratorischen Zweig des Beatmungsgerätes sicherzustellen.

Die Gassteuerungsvorrichtung ist eingerichtet, bei einer Blockade bzw. Störung des exspiratorischen Zweiges den zweiten Gaskanal derart zu steuern, dass der Gasstrom des ersten Gaskanals über die Öffnung ausströmt bzw. an dem Rückschlagventil über einen Umgehungskanal vorbeiströmt. Der Gasstrom des ersten Gaskanals setzt sich dabei in der Regel aus einem Einatemgas und/oder einem Ausatemgas zusammen. Insbesondere bei einer Blockade/Störung des exspiratorischen Zweiges kann das Ausatemgas eines Patienten über den inspiratorischen Zweig/den ersten Gaskanal, bis hin zu dem Rückschlagventil zurückgeführt werden. Daher ist die Öffnung des ersten Gaskanals vorteilhafterweise im Bereich des Rückschlagventils in Inspirationsflussrichtung dem Rückschlagventil nachgeschaltet angeordnet.

Die Gassteuerungsvorrichtung ist eingerichtet, den Druck in dem zweiten Gaskanal zu steuern. Zu steuern kann dabei bedeuten, dass die Schalteinrichtung eingerichtet sein kann, den zweiten Gaskanal freizugeben oder zu sperren. In einer alternativen Ausführungsform kann die Schalteinrichtung kontinuierlich stufenlos steuerbar sein. Basierend auf der Steuerung des Gasstroms des zweiten Gaskanals, insbesondere der Freigabe oder Sperrung des zweiten Gaskanals, kann der Gasstrom des ersten Gaskanals (das Ausatemgas/ der Ausatem-Gasstrom) an dem Rückschlagventil vorbeigeführt werden. Der Gasstrom des ersten Gaskanals wird über einen Umgehungskanal an dem Rückschlagventil vorbeigeführt. Der in den Umgehungskanal umgelenkte Gasstrom kann erneut dem ersten Gaskanal, vorteilhafterweise in einem Bereich der in Inspirationsflussrichtung des Gasstroms dem Rückschlagventil vorgeschaltet liegt, zugeführt werden oder über den Umgehungskanal oder einen separaten Zweig, der als inspiratorischer oder exspiratorischer Zweig eingerichtet sein kann, abgeführt werden.

Die Gassteuerungsvorrichtung kann dabei eingerichtet sein, basierend auf einem erfassten Volumen, einem erfassten Durchfluss oder einem erfassten Druck eines Gasstroms steuerbar zu sein.

insbesondere kann die Gassteuerungsvorrichtung mindestens einen Sensor umfassen, wobei die Schalteinrichtung eingerichtet sein kann, basierend auf einem durch den mindestens einen Sensor erfassten Parameter steuerbar zu sein.

Der mindestens eine Sensor der Gassteuerungsvorrichtung beispielsweise als Teil der Schalteinrichtung kann eingerichtet sein, mindestens einen Atmungsparameter, Atemgasparameter und/oder einen anderen Parameter aus Ausgangssignalen zu erfassen. Dabei können ein oder mehrere erfasste Parameter eine Funktion bilden, die sich beispielsweise auf Messungen von einem oder mehreren der folgenden beziehen: (Spitzen-) Durchfluss, Durchflussrate, (Tidal-) Volumen, Druck, Temperatur, Feuchtigkeit, Geschwindigkeit, Beschleunigung, Gaszusammensetzung (z. B. Konzentration(en) von einem oder mehreren Bestandteilen), thermische Energiedissipation, (beabsichtigte) Gasleckage und/oder andere Messungen in Bezug auf den Atemgasfluss.

Atmungsparameter können beispielsweise aus Gasparametern und/oder anderen Ausgangssignalen abgeleitet werden, wobei ein oder mehrere Atmungsparameter eine oder mehrere der folgenden sein können: Atmungsfrequenz, Atmungsdauer, Einatmungszeit oder -dauer, Ausatmungszeit oder -periode, Form der Atmungsflusskurve, Übergangszeit von der Einatmung zur Ausatmung und/oder umgekehrt, Übergangszeit von der (Spitze-) Inhalationsflussrate zu (Spitzen-) Exspirationsflussrate und/oder umgekehrt, Atmungsdruckkurvenform, maximaler proximaler Druckabfall (pro Atmungszyklus und/oder Phase), Anteil an eingeatmetem Sauerstoff und/oder andere Atmungsparameter.

Die erfindungsgemäße Gassteuerungsvorrichtung ermöglicht es einem Patienten auch bei einer Blockade/Störung des exspiratorischen Zweiges des Beatmungsgerätes ausatmen zu können.

Die Gassteuerungsvorrichtung beispielsweise über die Schalteinrichtung ist eingerichtet, den Gasstrom des zweiten Gaskanals zu steuern und eine Vorbeiführung des Gasstroms des ersten Gaskanals um das Rückschlagventil durch die Öffnung zu ermöglichen. Durch die Steuerung des Gasstroms des zweiten Gaskanals kann die Öffnung, auf der die Dichtung aufliegt, die durch den Gasstrom/Druck des zweiten Gaskanals abgedichtet wird, geöffnet werden. Der Gasstrom des ersten Gaskanals kann aus der Öffnung austreten. Bei einer Sperrung des zweiten Gaskanals ändert sich das Druckverhältnis im Bereich der Dichtung, sodass ein Gasdruck des Gasstroms des ersten Gaskanals größer ist als ein Gasdruck des zweiten Gaskanals. In der Regel ist der Gasdruck des gesperrten zweiten Gaskanals gleich null.

In der Regel ist der Umgehungskanal ringförmig um die Öffnung des ersten Gaskanals ausgebildet. Ringförmig kann auch die Öffnung umschließend bedeuten. Die ringförmige Ausgestaltung bietet den Vorteil, dass Gas in einem Winkel von bis zu 360° aus der Öffnung strömen kann. Dadurch kann eine entsprechend große Menge an exspiratorischem Ausatemgas gleichzeitig über die Öffnung des ersten Gaskanals abgeführt werden. Vorteilhafterweise ist der Umgehungskanal eingerichtet, den über die Öffnung ausgegebenen Gasstrom zu bündeln und in eine Richtung abzuführen.

In Ausgestaltung ist die Gassteuerungsvorrichtung beispielsweise über die Schalteinrichtung eingerichtet, den zweiten Gaskanal freizugeben oder zu sperren. Beispielsweise ist die Schalteinrichtung eingerichtet, den zweiten Gaskanal freizugeben, wodurch auf die Dichtung, die auf der Öffnung aufliegt, ein Gasstrom/Druck beaufschlagt werden kann. Durch die Beaufschlagung der Dichtung mit einem Gasstrom/Druck ist die Dichtung auf die Öffnung andrückbar. Die Schalteinrichtung kann auch eingerichtet sein den zweiten Gaskanal zu sperren, sodass kein Gasstrom/Druck auf die Dichtung, die auf der Öffnung aufliegt, beaufschlagt werden kann. In diesem Fall liegt die Dichtung locker auf der Öffnung auf. Bei dieser Ausgestaltung kann ein Gasstrom des ersten Gaskanals durch die lose/locker auf der Öffnung aufliegende Dichtung in einen Umgehungskanal strömen.

Vorteilhafterweise ist die Gassteuerungsvorrichtung beispielsweise über die Schalteinrichtung eingerichtet, den zweiten Gaskanal bei einer Blockade/Störung des exspiratorischen Zweiges zu sperren. Zu sperren bedeutet, dass die Schalteinrichtung keinen Gasstrom/Druck in dem zweiten Gaskanal bereitstellt bzw. diesen verschließt. Durch die Nicht-Beaufschlagung der Dichtung mit einem Gasstrom des zweiten Gaskanals ist der Gasstrom des ersten Gaskanals ausreichend, um die Dichtung anzuheben und den Gasstrom aus dem ersten Gaskanal in den Umgehungskanal zu überführen.

Typischerweise ist die Gassteuerungsvorrichtung beispielsweise über die Schalteinrichtung eingerichtet, den zweiten Gaskanal bei einer Verwendung des Beatmungsgerätes in einem Ventilsystem freizugeben, sodass der zweite Gaskanal einen Gasstrom führen kann. Bei der Verwendung in einem Ventilsystem ist/sind der exspiratorische und/oder der inspiratorische Zweig des Beatmungsgerätes störungsfrei ist/sind und die Öffnung der Gassteuerungsvorrichtung wird durch einen Druck des zweiten Gaskanals, der in der Regel als Druckkanal ausgestaltet ist, verschlossen.

Freigeben bedeutet, dass die Gassteuerungsvorrichtung beispielsweise über die Schalteinrichtung den zweiten Gaskanal für das Durchströmen mit einem Gasstrom öffnet und somit die Dichtung, die auf der Öffnung aufliegt, mit einem Gasstrom/Druck beaufschlagt. In der Regel wird der Gasstrom des zweiten Gaskanals mit einem Druck von 1mbar bis 100mbar beaufschlagt. Durch den Druck wird die Dichtung auf die Öffnung gedrückt und abgedichtet.

Die Gassteuerungsvorrichtung ist eingerichtet, bei einem Normalbetrieb des Beatmungsgerätes den Gasstrom aus dem ersten Gaskanal mittels des Gasstroms des zweiten Gaskanals, der über einen Anschluss der Verschlusskappe auf die Dichtung der Gassteuerungsvorrichtung geleitet wird, die Dichtung abzudichten.

So kann bei einem Normalbetrieb/einer Verwendung in einem Ventilschlauchsystem die Funktion des Rückschlagventils erhalten bleiben.

Die Gassteuerungsvorrichtung ist eingerichtet, bei einem störungsfreien Betriebszustand des Beatmungsgerätes beispielsweise bei einem störungsfreien exspiratorischen Zweig einen Druck über den zweiten Gaskanal auf die Dichtmembran aufzubringen und die Öffnung der Gassteuerungsvorrichtung dadurch zu verschließen.

Die Gassteuerungsvorrichtung ist eingerichtet, bei einer Blockade/Störung des exspiratorischen Zweiges den zweiten Gaskanal zu sperren. Dadurch liegt kein Druck auf der Dichtmembran auf und die Öffnung ist durch einen Gasdruck des Gasstroms des ersten Gaskanals öffnenbar. Der Gasstrom, der über die Öffnung austritt, kann erneut dem ersten Gaskanal zugeführt werden oder über einen separaten Zweig abführbar sein.

In einer Ausgestaltung kann die Schalteinrichtung (als Teil der Gassteuerungsvorrichtung) eingerichtet sein, basierend auf einer zeitgesteuerten Schaltung einer Exspiration und/oder einer Inspiration steuerbar zu sein.

In einer alternativen Ausgestaltung kann die Schalteinrichtung (als Teil der Gassteuerungsvorrichtung) eingerichtet sein, basierend auf einem Parameter des ersten Gaskanals steuerbar zu sein. Beispielsweise kann mittels eines Sensors mindestens ein Parameter des Gasstroms in dem ersten Gaskanal erfasst werden. Beispielsweise kann mittels eines Drucksensors ein Druck innerhalb des ersten Gaskanals erfasst werden. Die Schalteinrichtung ist dann eingerichtet in Abhängigkeit des erfassten Drucks des Gasstroms in dem ersten Gaskanal steuerbar zu sein. Beispielsweise kann die Schalteinrichtung in der alternativen Ausgestaltung eingerichtet sein, den zweiten Gaskanal in Abhängigkeit der Erreichung eines vorbestimmten Schwellenwertes freizugeben oder zu sperren. Ein solcher Schwellenwert kann beispielsweise ein Wert sein, der über dem durchschnittlich erfassten Druck im ersten Gaskanal liegt. Bei einer Schwellwertüberschreitung kann die Schalteinrichtung eingerichtet sein, den zweiten Gaskanal zu sperren. Sinkt der Druck im ersten Gaskanal unter den Schwellenwert kann die Schalteinrichtung den zweiten Gaskanal freigeben.

In einer weiteren alternativen Ausführungsform kann mittels eines Sensors beispielsweise auch die Durchflussrate oder ein Volumen des Gasstroms des ersten Gaskanals erfasst werden. Die Schalteinrichtung kann eingerichtet sein, basierend auf der ermittelten Flussrate oder dem erfassten Volumen des Gasstroms steuerbar zu sein. Auch kann die Schalteinrichtung eingerichtet sein basierend auf weiteren Parametern des Gasstroms des ersten Gaskanals steuerbar zu sein.

In einer weiteren alternativen Ausführungsform kann die Schalteinrichtung (als Teil der Gassteuerungsvorrichtung) zudem variabel schaltbar eingerichtet sein, wobei die Schalteinrichtung eingerichtet sein kann, einen Druck des Gasstroms in dem zweiten Gaskanal zwischen 0mbar und 1bar, insbesondere zwischen 0mbar und 100mbar stufenlos zu steuern. Die Schalteinrichtung kann auch eingerichtet sein, den Gasstrom/Druck in dem zweiten Gaskanal kontinuierlich über einen vorbestimmten Zeitraum auf einen vorbestimmten Wert zu erhöhen.

Die Dichtung für einen Teil der Gassteuerungsvorrichtung, insbesondere für ein Ventil ist ausgebildet, bei einem gesperrten zweiten Gaskanal einen Durchfluss des Gasstroms des ersten Gaskanals durch die Öffnung zu ermöglichen. Die Dichtung dichtet den Umgehungskanal gegen die Öffnung ab. Die Dichtung weist vorteilhafterweise eine Form auf, die die Dichtung in einem Gasleeren Zustand des ersten und des zweiten Gaskanals auf der Öffnung ablegt und hält. Die Dichtung weist ein Auflageflächenverhältnis zwischen $4/5$ und $2/3$ auf. Die Dichtung ist derart dimensioniert, dass diese durch einen Gasstrom des ersten Gaskanals angehoben werden kann, wenn die Dichtung nicht durch einen Gasstrom des zweiten Gaskanals mit einem Gasstrom/Druck beaufschlagt wird. Ferner umfasst die Dichtung in der Regel einen umlaufenden, verstärkten Rand, der eingerichtet ist, auf dem Rand des Umgehungskanals aufzuliegen. Der umlaufende, verstärkte Rand, kann eine Verrastungsstruktur oder Haltestruktur aufweisen, die es erleichtert, die Dichtung auf dem Rand des Umgehungskanals zu halten. Beispielsweise kann der umlaufende, verstärkte Rand eine Kante aufweisen, die an eine Hervorhebung des Randes des Umgehungskanals anliegend ausgebildet ist.

In Ausgestaltung ist die Dichtung auf der Öffnung aufliegend angeordnet und umfasst ein Gewicht. Das Gewicht, welches vorzugsweise in die Dichtung integriert ausgebildet ist, unterstützt die Positionierung der Dichtung auf der Öffnung. In der Regel weist die Dichtung in dem Bereich, in dem das Gewicht integral ausgebildet ist, im Vergleich zu den umgebenden Bereichen der Dichtung eine höhere Materialstärke auf. Die Dichtung ist typischerweise kreisförmig ausgebildet. Die Dichtung kann alternativ rechteckig, quadratisch oder oval ausgebildet sein oder eine andere geometrische Form aufweisen. Die Dichtung ist in der Regel korrespondierend zu der Form der Öffnung und/oder zu der Form des die Öffnung umgebenden Umgehungskanals ausgebildet. Zwischen dem Rand des Umgehungskanals und dem Gewicht kann die Dichtung eine Wölbung ausbilden, die in Richtung des ersten Gaskanals weist und einem Verrutschen der Dichtung vorbeugt.

In einer Weiterbildung der Erfindung ist das Gewicht der Dichtung ringförmig in der Mitte der Dichtung ausgebildet. In der Mitte bedeutet dabei im Zentrum der kreisförmigen Dichtung. In der Regel ist das ringförmige Gewicht unterlegscheibenförmig ausgebildet. Das Gewicht kann jedoch ebenfalls eine rechteckige oder ovale Form aufweisen. Durch die ringförmige Ausgestaltung kann die Dichtung auf einem die Öffnung umlaufend umgebenden Rand der Öffnung abgelegt werden. Die Dichtung kann auch nur bereichsweise auf dem Rand der Öffnung aufliegen. In der Regel ist die Form des Gewichts korrespondierend zur Form der Öffnung bzw. zur Form des Randes der Öffnung ausgebildet. Das Gewicht beschwert die Dichtung, wodurch die Dichtung locker auf dem Rand der Öffnung gehalten wird. Optional kann das Gewicht rechteckig ausgebildet sein oder eine andere geometrische Form aufweisen. Zudem kann das Gewicht in einem anderen Bereich der Dichtung angeordnet sein.

Die Dichtung ist aus einem elastomeren Material ausgebildet und das Gewicht ist aus einem Metall ausgebildet. Die Dichtung ist in der Regel aus einem elastomeren Material mit einer Härte im Bereich zwischen 15 bis 25 Shore A, insbesondere zwischen 18 und 22 Shore A ausgebildet. Beispielsweise ist die Dichtung aus einem Silikon gefertigt. Alternativ kann das Gewicht aus einem anderen Material, beispielsweise einem Hartplastik oder einer Kombination aus Metall und Kunststoff, ausgebildet sein.

In einer weiteren Weiterbildung der Erfindung weist die Dichtung eine Ausprägung auf, die im Bereich des Gewichts in Richtung des ersten Gaskanals weisend ausgebildet ist. Die Ausprägung kann als kegelförmige Hervorhebung ausgestaltet sein, deren Spitze sich von der Öffnung in den ersten Gaskanal hineinweisend erstreckt. Beispielsweise kann die Ausprägung in der durch die unterlegscheibenförmige Ausgestaltung bedingten Ausnehmung des Gewichts ausgebildet sein. Die Ausprägung unterstützt die Dichtung auf der Öffnung des ersten Gaskanals zu halten. Die Ausprägung kann optional in einer beliebigen Form ausgebildet sein, die die Dichtung in ihrer Position auf der Öffnung hält. In einer alternativen Ausführungsform kann die Dichtung ausprägungsfrei ausgebildet sein.

In einer vorteilhaften Weiterbildung der Erfindung ist die Verschlusskappe eingerichtet, auf die Öffnung aufsetzbar zu sein. In der Regel ist die Verschlusskappe auf die Öffnung aufklippbar. Dies bietet den Vorteil, dass die Verschlusskappe beim Aufsetzen auf die Dichtung, die auf der Öffnung aufliegt, diese in ihrer Position nicht verschiebt. Um auf die Öffnung aufklippbar zu sein, weist die Verschlusskappe in der Regel mindestens zwei Fortsätze auf, die typischerweise Widerhaken aufweisen, die in Ausnehmungen, die an einem Außenbereich der Öffnung ausgebildet sind, eingreifen. Alternativ kann die Verschlusskappe der Gassteuerungsvorrichtung mit einem Bajonettverschluss versehen sein. Optional kann die Verschlusskappe mit alternativen Verrastungselementen auf der Öffnung befestigt werden.

In einer weiteren vorteilhaften Weiterbildung der Erfindung umfasst die Verschlusskappe einen Anschluss, der mit dem zweiten Gaskanal verbindbar ist. Die Form des Anschlusses ist in der Regel korrespondierend zu der Form des zweiten Gaskanals ausgebildet. Alternativ sind der Anschluss und der Gaskanal durch einen Adapter verbindbar. Der Anschluss ist eingerichtet, den durch die Schalteinrichtung bereitgestellten Gasstrom/Druck des zweiten Gaskanals auf die Dichtung zu führen. Der Anschluss ist in der Regel mit der Verschlusskappe verbunden ausgebildet. Dabei ist der Anschluss typischerweise derart mit der Verschlusskappe verbunden, dass ein Gasstrom vertikal und mittig auf die Dichtung aufbringbar ist. Alternativ kann der Anschluss auch seitlich an der Verschlusskappe angeordnet sein. Der Anschluss, der ebenfalls als Kanal ausgebildet sein kann, weist beispielsweise einen Durchmesser zwischen 0,1mm bis 0,5 mm auf.

Erfindungsgemäß ist die Schalteinrichtung (als Teil der Gassteuerungsvorrichtung) eingerichtet, bei einem Normalbetrieb des Beatmungsgerätes den zweiten Gaskanal freizugeben und einen Gasstrom/Druck über den zweiten Gaskanal und den Anschluss auf die Dichtung zu leiten und die Öffnung zu schließen. Bei einem Normalbetrieb des Beatmungsgerätes liegt keine Störung/Blockade eines exspiratorischen oder inspiratorischen Zweiges des Beatmungsgerätes vor. Die Schalteinrichtung ist eingerichtet, den Gasstrom/Druck des zweiten Gaskanals zu steuern. In der Regel wird der Gasstrom in dem zweiten Gaskanal mit einem Gasstrom/Druck von 1mbar bis 1bar, insbesondere zwischen 10mbar und 150mbar, beaufschlagt. Dadurch wird die Dichtung auf die Öffnung des ersten Gaskanals abgedichtet, so dass der Gasstrom des ersten Gaskanals nicht über die Öffnung entweichen kann.

Erfindungsgemäß ist die Schalteinrichtung (als Teil der Gassteuerungsvorrichtung) eingerichtet, bei einer Blockade/Störung des exspiratorischen Zweiges den zweiten Gaskanal zu sperren. Durch die Sperrung des zweiten Gaskanals kommt der Gasstrom/Druck des zweiten Gaskanals zum Erliegen. Die Gassteuerungsvorrichtung kann den Gasstrom des ersten Gaskanals über die Öffnung ausleiten. Bei einer Sperrung des zweiten Gaskanals durch die Schalteinrichtung ist der Gasstrom/Druck in dem ersten Gaskanal größer als im zweiten Gaskanal. Dadurch kann der Gasstrom des ersten Gaskanals die Dichtung, die auf der Öffnung aufliegt, anheben und den Gasstrom in den Umgehungskanal führen und das Rückschlagventil umgehen. Die Dichtung wird derart angehoben, dass die Dichtung im Bereich der Begrenzung der Dichtung durch die Verschlusskappe an den Rand des Umgehungskanals angedrückt/gehalten wird. Sobald der zweite Gaskanal gesperrt ist und kein Gasstrom/Druck mehr auf die Dichtung aufgebracht wird, wird das Gewicht der Dichtung durch den Gasstrom des ersten Gaskanals angehoben, sodass die Dichtung, die auf dem Rand der Öffnung aufliegt angehoben wird, wodurch der Gasstrom von dem ersten Gaskanal über die Öffnung in den Umgehungskanal strömen kann.

Die vorliegende Erfindung umfasst ferner auch eine Dichtung für einen Teil der Gassteuerungsvorrichtung für ein Beatmungsgerät umfassend einen ersten Gaskanal mit einem Rückschlagventil, einen zweiten Gaskanal und eine Schalteinrichtung, wobei der erste Gaskanal eine Öffnung zum Ausströmen von Gas mit einer Dichtung und einer Verschlusskappe aufweist.

Erfindungsgemäß weist die Dichtung ein Gewicht auf und ist eingerichtet, bei einem gesperrten zweiten Gaskanal einem Gasstrom des ersten Gaskanals einen Durchlass durch die Öffnung zu ermöglichen. Die Dichtung ist in der Regel kreisförmig ausgebildet. Alternativ kann die Dichtung jedoch auch rechteckig ausgebildet sein oder eine andere geometrische Form aufweisen. Typischerweise ist die Dichtung aus einem elastomeren Material, beispielsweise Silikon, ausgebildet. Die Dichtung weist in der Regel eine Härte zwischen 15 bis 25 Shore A, insbesondere zwischen 18 und 22 Shore A auf. Das Gewicht kann in einer alternativen Ausgestaltung in einem anderen Bereich der Dichtung ausgebildet sein.

In Ausgestaltung weist die Dichtung einen zumindest teilweise umlaufenden Rand auf, der eingerichtet ist, in eine Ausnehmung der Gassteuerungsvorrichtung einzugreifen. Der umlaufende Rand ist vorzugsweise verstärkt ausgebildet und kann eine Ausprägung aufweisen, die in eine Ausnehmung am Rand des Umgehungskanals eingreift. Der umlaufende Rand der Dichtung dient zum einen der Abdichtung und zum anderen der Positionierung der Dichtung auf dem Rand des Umgehungskanals. Er wird durch die Verschlusskappe auf den Rand des Umgehungskanals gedrückt/gehalten/abgedichtet. Der Rand des Umgehungskanals kann eine Hervorhebung aufweisen, die eingerichtet ist, an die Dichtung derart anliegend zu sein, dass eine Kante der Dichtung mit der Hervorhebung in Eingriff kommt.

Die vorliegende Erfindung betrifft auch eine Gassteuerungsvorrichtung die Gassteuerungsvorrichtung als elektrische und/oder pneumatische Steuereinheit in einem Beatmungsgerät ausgebildet und eingerichtet ist, einen unter Druck stehenden Gasstrom in einer ersten Richtung passieren zu lassen und einen unter Druck stehenden Gasstrom in einer zweiten Richtung zu sperren und einen Gasstrom vom dem ersten Gaskanal zu dem Umgehungskanal zu sperren und/oder zumindest zeitweise zu ermöglichen, wobei die Gassteuerungsvorrichtung eingerichtet ist, einen Gasstrom von dem ersten Gaskanal zu dem Umgehungskanal zu sperren und/oder zumindest zeitweise zu ermöglichen.

Die Gassteuerungsvorrichtung kann als elektrische und/oder pneumatische Steuereinheit ausgebildet sein, die unterschiedliche Aktoren und Sensoren umfasst, beispielsweise zumindest die Schalteinrichtungen, Ventile und die Gasquelle und diese koordiniert im Sinne der Erfindung ansteuert.

Die Gassteuerungsvorrichtung kann als elektrische und/oder pneumatische Steuereinheit ausgebildet und eingerichtet sein, einen unter Druck stehenden Gasstrom in einer ersten Richtung passieren zu lassen und einen unter Druck stehenden Gasstrom in einer zweiten Richtung zu sperren und einen Gasstrom vom dem ersten Gaskanal zu dem Umgehungskanal zu sperren und/oder zumindest zeitweise zu ermöglichen, wobei eine Gassteuerungsvorrichtung (10) eingerichtet ist, einen Gasstrom von dem ersten Gaskanal zu dem Umgehungskanal zu sperren und/oder zumindest zeitweise zu ermöglichen.

Die vorliegende Erfindung umfasst des Weiteren eine Verschlusskappe für eine Gassteuerungsvorrichtung für ein Beatmungsgerät umfassend einen ersten Gaskanal mit einem Rückschlagventil, einen zweiten Gaskanal und eine Schalteinrichtung, wobei der erste Gaskanal eine Öffnung zum Ausströmen von Gas mit einer Dichtung und einer Verschlusskappe aufweist.

Erfindungsgemäß weist die Verschlusskappe zumindest bereichsweise Fortsätze auf, die Widerhaken ausbilden und eingerichtet sind, in Ausnehmungen an der Gassteuerungsvorrichtung einzugreifen. Alternativ kann die Verschlusskappe einen Bajonettverschluss umfassen, der eingerichtet ist in Ausnehmungen an der Öffnung einzugreifen.

Die vorliegende Erfindung umfasst des Weiteren ein Beatmungsgerät umfassend eine Gassteuerungsvorrichtung gemäß mindestens einem der vorgenannten Merkmale.

Gas kann im Sinne der Erfindung jedes atembare Gas oder Gasgemisch sein. Insbesondere Luft, Sauerstoff, Atemgas der Exspiration oder Atemgas der Inspiration.

Im Folgenden werden anhand von stark vereinfachten schematischen Darstellungen bevorzugte Ausführungsbeispiele der Erfindung näher erläutert. Es zeigt:

Fig. 1a     eine schematische Ansicht einer erfindungsgemäßen Gassteuerungsvorrichtung,

Fig. 1b     eine schematische Ansicht einer erfindungsgemäßen Gassteuerungsvorrichtung, wobei die Gassteuerungsvorrichtung hier nur teilweise dargestellt nämlich als Schalteinrichtung oder als Ventilblock

Fig. 2     eine schematische Explosionsansicht der in Figur 1b gezeigten Gassteuerungsvorrichtung,

Fig. 3     einen Längsschnitt durch die in den Figuren 1b und 2 gezeigten Gassteuerungsvorrichtung,

Fig. 4a     eine Seitenansicht der in den Figuren 1b bis 3 gezeigten Gassteuerungsvorrichtung,

Fig. 4b     eine Draufsicht auf eine erfindungsgemäße Verschlusskappe der in den Figuren 1b bis 4a gezeigten Gassteuerungsvorrichtung,

Fig. 4c     eine Seitenansicht auf ein Rückschlagventil der in den Figuren 1b bis 4b gezeigten Gassteuerungsvorrichtung.

In den Figuren weisen dieselben konstruktiven Elemente jeweils dieselben Bezugsziffern auf.

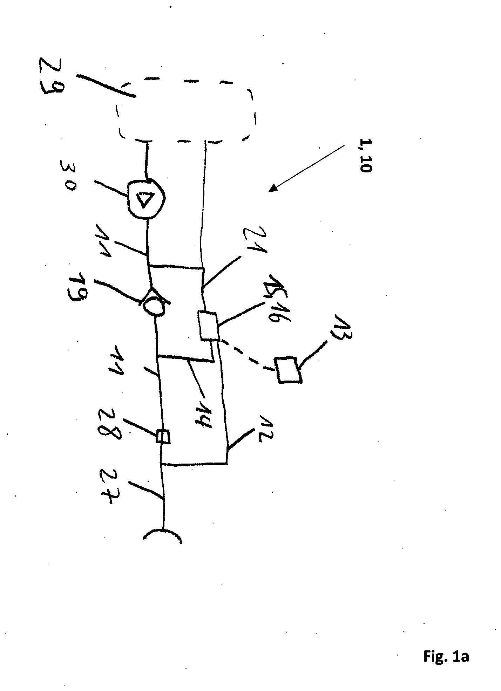

Figur 1a zeigt eine schematische Ansicht einer erfindungsgemäßen Gassteuerungsvorrichtung 10 für ein Beatmungsgerät 1. Beatmungsgeräte 1 weisen in der Regel einen inspiratorischen Zweig, der für die Zuführung von Atemgas zu einem Patienten vorgesehen ist, und einen exspiratorischen Zweig auf, der zur Abführung von durch den Patienten ausgeatmetem Atemgas dient. Die Gassteuerungsvorrichtung (10) kann als elektrische und/oder pneumatische Steuereinheit ausgebildet und eingerichtet sein, einen unter Druck stehenden Gasstrom in einer ersten Richtung (25) passieren zu lassen und einen unter Druck stehenden Gasstrom in einer zweiten Richtung (26) zu sperren und einen Gasstrom vom dem ersten Gaskanal (11) zu dem Umgehungskanal (21) zu sperren und/oder zumindest zeitweise zu ermöglichen, wobei eine Gassteuerungsvorrichtung (10) eingerichtet ist, einen Gasstrom von dem ersten Gaskanal 11 zu dem Umgehungskanal (21) zu sperren und/oder zumindest zeitweise zu ermöglichen.

Die in der Figur 1a gezeigte erfindungsgemäße Gassteuerungsvorrichtung 10 ist zur Verwendung in einem Beatmungsgerät 1 oder als Teil eines Beatmungsgerätes vorgesehen und weist einen ersten Gaskanal 11 mit einem ersten Ventil (19) auf, wobei der erste Gaskanal (11) und das Ventil (19) eingerichtet sind, einen unter Druck stehenden Gasstrom in einer ersten Richtung (25) passieren zu lassen und einen unter Druck stehenden Gasstrom in einer zweiten Richtung (26) zu sperren, wobei eine Öffnung (14) von dem ersten Gaskanal 11 abzweigt und zu einem Umgehungskanal (21), für das Ventil (19), führt, wobei ein zweites Ventil (15, 16) eingerichtet ist einen Gasstrom vom dem ersten Gaskanal (11) zu dem Umgehungskanal (21) zu sperren und/oder zumindest zeitweise zu ermöglichen, wobei eine Schalteinrichtung (13) eingerichtet ist das zweite Ventil (15, 16) zu steuern, einen Gasstrom vom dem ersten Gaskanal 11 zu dem Umgehungskanal (21) zu sperren und/oder zumindest zeitweise zu ermöglichen.

Die Schalteinrichtung umfasst hier beispielsweise den Ventilblock 13 mit ersten 19 und zweitem Ventil, dem ersten 11 und zweiten Gasweg und der Umgehung 14, 21. Optional umfasst die Schalteinrichtung 13 auch die pneumatische oder elektronische Steuerung für die Komponenten des Ventilblocks.

Eine Gas- oder Atemgasquelle 30, beispielsweise ein Gebläse, befördert Gas oder Atemgas, beispielsweise angesaugt aus der Umgebung 29, entlang dem ersten Gaskanal (11) für die Inspiration in der ersten Richtung (25) zu dem Schlauchstutzen (28) und dem Beatmungsschlauch (27). Dabei passiert der Gasstrom das geöffnete Rückschlagventil (19).

Eine Exspiration des Patienten sorgt für einen Atemgasstrom in einer zweiten Richtung (26) durch den Beatmungsschlauch, den Stutzen (28) und den ersten Gaskanal (11) bis zu dem Rückschlagventil (19). Dieses ist ein pneumatisch betätigtes Rückschlagventil (19), welches durch die Kraft des unter Druck stehenden Gasstroms der Exspiration, in der zweiten Richtung (26), betätigt wird und so das Rückschlagventil (19) nicht passieren kann. Da die Öffnung (14) mit dem ersten Gaskanal (11) in Verbindung steht, breitet sich der unter Druck stehende Gasstrom der Exspiration, in der zweiten Richtung (26), auch in die Öffnung aus und gelangt bis zu dem Ventil (15, 16). Sofern die Schalteinrichtung (13) das Ventil (15,16) öffnet, strömt der unter Druck stehende Gasstrom der Exspiration weiter entlang der Öffnung und den Umgehungskanal (21) entlang in den ersten Gaskanal und vor dort zumindest teilweise in die Umgebung.

Das Ventil (15, 16) kann als pneumatisches Ventil ausgeführt sein. In diesem Fall kann eine Dichtung (15) des Ventils die Gasleitende Verbindung zwischen Öffnung 14 und Umgebungskanal verschließen oder öffnen. Der Steuerimpuls für die Dichtung (15) des Ventils kommt von einem zweiten Gaskanal (12), der einen pneumatischen Steuerdruck auf die Oberfläche der Dichtung 15 leitet. Ist die Dichtung mit dem Steuerdruck beaufschlagt, so legt sie sich dichtend an den Rand 22 der Öffnung 14 und verhindert so einen Gasstrom von der Öffnung in den Umgehungskanal.

Der zweite Gaskanal kann dazu aus dem Beatmungsschlauch oder. dem Gaskanal (11) pneumatisch gespeist werden. Ebenfalls ist eine andere Druckgasquelle, wie ein Steuergebläse, denkbar. Die Schalteinrichtung 13 öffnet oder verschließt die pneumatische Verbindung zwischen dem zweiten Gaskanal 12 und dem Beatmungsschlauch oder dem Gaskanal (11) zumindest zeitweise.

Das Ventil (15, 16) kann als Schalt-Ventil ausgeführt sein. In diesem Fall öffnet oder verschließt eine Aktivierung des Ventils, durch die Schalteiririchtung 13, den Gasstrom von der Öffnung 14 in den Umgehungskanal. In diesem Fall ist ein zweiter Gaskanal entbehrlich. Beatmungsgeräte weisen in der Regel einen inspiratorischen Zweig, der für die Zuführung von Atemgas zu einem Patienten vorgesehen ist, und einen exspiratorischen Zweig auf, der zur Abführung von durch den Patienten ausgeatmetem Atemgas dient.

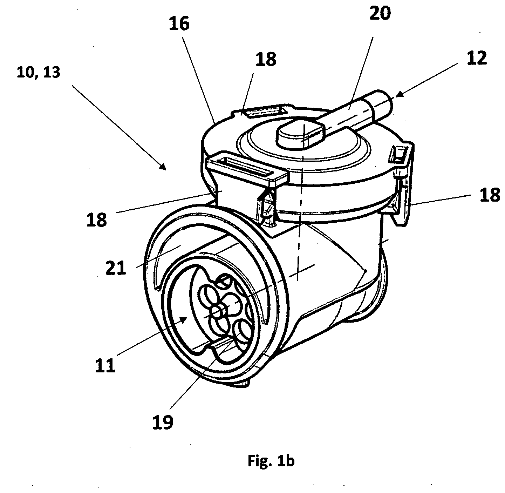

Figur 1b zeigt eine schematische Ansicht einer erfindungsgemäßen

Gassteuerungsvorrichtung 10 für ein Beatmungsgerät. Die Gassteuerungsvorrichtung (10) ist hier nur teilweise dargestellt nämlich als Schalteinrichtung 13 oder als Ventilblock und umfasst einen ersten Gaskanal (11) mit einem ersten Ventil (19), wobei der erste Gaskanal (11) und das Ventil (19) eingerichtet sind, einen unter Druck stehenden Gasstrom in einer ersten Richtung (25) passieren zu lassen und einen unter Druck stehenden Gasstrom in einer zweiten Richtung (26) zu sperren, wobei eine Öffnung (14) von dem ersten Gaskanal 11 abzweigt und zu einem Umgehungskanal (21), für das Ventil (19), führt, und ein zweites Ventil (15, 16) um den Umgehungskanal (21) zu öffnen oder zu schließen.

Die in der Figur 1 und 1b gezeigte erfindungsgemäße Gassteuerungsvorrichtung 10 ist zur Verwendung in einem Beatmungsgerät vorgesehen und weist einen ersten Gaskanal 11 (inspiratorischen Zweig) auf, der einen Gasstrom in Richtung eines Patienten führt. Der erste Gaskanal 11 umfasst ein Rückschlagventil 19, sowie eine - nicht gezeigte - Öffnung mit einer - nicht gezeigten- Dichtung. Die Öffnung und die Dichtung sind in der Figur 1 von einer Verschlusskappe 16 verdeckt. Das Rückschlagventil 19 verhindert eine Rückführung eines von dem Patienten kommenden, durch die Ausatmung verunreinigten, Atemgases/Gasstroms.

Zudem weist die Gassteuerungsvorrichtung 10 einen zweiten Gaskanal 12 auf, der einen Gasstrom zu der -nicht gezeigten- Öffnung führt. Der zweite Gaskanal 12 ist über einen Anschluss 20 mit der Verschlusskappe 16 verbunden, die auf die Öffnung des ersten Gaskanals aufgeklippt ist. Der zweite Gaskanal 12 verläuft über den Anschluss 20 zu der Verschlusskappe 16 und wird durch eine -nicht gezeigte- Schalteinrichtung gesteuert. Der zweite Gaskanal 12 kann von einem Gasstrom durchflossen werden.

Die - nicht gezeigte - Schalteinrichtung kann den zweiten Gaskanal 12 freigeben oder sperren. Dabei gibt die Schalteinrichtung den zweiten Gaskanal 12 für einen Gasstrom, bzw. einen Gasdruck bzw. ein Gasstromvolumen frei. Bei einem Normalbetrieb des Beatmungsgerätes ist der zweite Gaskanal 12 freigegeben, so dass der Gasstrom bzw. Druck über den Anschluss 20 auf die - nicht gezeigte - Dichtung innerhalb der Verschlusskappe 16 führbar ist. Durch den Gasstrom/Druck des zweiten Gaskanals 12 wird die Dichtung vollständig auf die Öffnung gedrückt und die Öffnung somit verschlossen. Dadurch wird bei einem Normalbetrieb des Beatmungsgerätes, bei dem keine Blockade vorliegt, ein Ausströmen eines inspiratorischen Gasstroms des ersten Gaskanals 11, der zu dem Patienten geführt werden soll, über die Öffnung in den Umgehungskanal 21 verhindert. Dies ist in diesem Fall nicht notwendig, da der exspiratorische Zweig blockadefrei ist, sodass die ausgeatmete AtemGas des Patienten über den exspiratorischen Zweig abgegeben werden kann.

Bei einer Blockade/Störung eines exspiratorischen Zweiges des Beatmungsgerätes, hingegen wird ein Ausatmen des Patienten verhindert oder erschwert. Durch das Rückschlagventil 19 kann der Patient zudem nicht über den inspiratorischen Zweig ausatmen. Bei einer Blockade/Störung des exspiratorischen Zweiges des Beatmungsgerätes sperrt die Schalteinrichtung den zweiten Gaskanal 12. Durch den gesperrten Gaskanal 12 kommt der Gasstrom des zweiten Gaskanals 12, der auf die Dichtung wirkt, zum Erliegen, wodurch der Gasstrom des ersten Gaskanals 11 die Dichtung, die auf der Öffnung aufliegt, anheben kann, sodass der Gasstrom des ersten Gaskanals über einen Umgehungskanal 21 um das Rückschlagventil herumgeführt wird. In diesem Fall ist somit der Gasstrom bzw. Druck des ersten Gaskanals 11 größer als der Gasstrom bzw. der Druck des zweiten Gaskanals 12, wodurch der Gasstrom des ersten Gaskanals 11 die Dichtung, die auf der Öffnung aufliegt, anheben kann. Die Dichtung ist derart dimensioniert und ausgebildet, dass der Gasstrom des ersten Gaskanals 11 bei einem gesperrten Gaskanal 12 diese anheben kann. Anheben bedeutet dabei, dass die Dichtung an ihrem umlaufenden Rand durch die Verschlusskappe 16 auf dem Rand des Umgehungskanals 21 umlaufend gehalten wird und in Richtung des Zentrums der kreisförmig ausgestalteten Dichtung durch den Gasstrom des ersten Gaskanals 11 hochgedrückt wird. Das Gewicht der Dichtung ist derart proportioniert, dass es die Dichtung bei einem Nachlassen des Gasstroms des zweiten Gaskanals 12 automatisch auf der Öffnung ablegt.

Der Patient kann somit auch bei einer Blockade/Störung des exspiratorischen Zweiges ausatmen.

Bei einem Leckagesystem sperrt die Schalteinrichtung den zweiten Gaskanal bzw. gibt keinen Druck auf die Dichtung. In diesem Fall ist das Schaltventil eingerichtet dauerhaft ein Ausströmen von Gas zu ermöglichen.

Bei einem Ventilsystem ist die Schalteinrichtung eingerichtet, entsprechend der zeitgesteuerten Exspiration des Beatmungsgerätes zu schalten. Somit ist die Schalteinrichtung eingerichtet, bei der Schaltung des Beatmungsgerätes auf eine Exspiration, den zweiten Gaskanal 12 zu sperren. Dadurch wird die Exspiration eines Patienten sichergestellt, da die Öffnung der Gassteuerungsvorrichtung während der Exspiration geöffnet ist und eine rückströmende Gas über die Öffnung entweichen kann.

Bei einem Normalbetrieb des exspiratorischen Zweiges des Beatmungsgerätes schaltet die Schalteinrichtung den zweiten Gaskanal 12 frei, wodurch ein Gasstrom in den zweiten Gaskanal 12 strömen kann und mit einem Druck beaufschlagt wird. Durch den Gasstrom in dem zweiten Gaskanal 12 wird die Dichtung auf der Öffnung abgedichtet, wodurch ein Ausströmen von inspiratorischer Gas durch die Öffnung während des Normalbetriebs verhindert wird

Die Verschlusskappe 16 umfasst in der vorliegenden Ausführungsform drei Fortsätze 18 mittels derer die Verschlusskappe 16 auf die Öffnung des ersten Gaskanals 11 aufklippbar ist. Zudem ist an der Verschlusskappe ein Anschluss 20 ausgebildet, mit dem der zweite Gaskanal 12 verbindbar ist. Der Anschluss 20 ist mittig auf der Verschlusskappe 16 angeordnet, so dass der Gasstrom/Druck über den Anschluss 20 und die Verschlusskappe 16 mittig und gleichmäßig auf die Dichtung aufbringbar ist.

Die in der Figur 1b gezeigte Gassteuerungsvorrichtung 10 ist somit bei einem Normalbetrieb eingerichtet, den Gasstrom in dem ersten Gaskanal 11 durch das Rückschlagventil 19 zu dem Patienten hin zu führen. Bei einem Normalbetrieb des exspiratorischen Zweiges wird die Öffnung des ersten Gaskanals 11 verschlossen und die Funktion des Rückschlagventils 19 bleibt bestehen. Bei einer Blockade/Störung des Rückschlagventils 19 umgeht die Gassteuerungsvorrichtung 10 das Rückschlagventil 19.

Die - nicht gezeigte - Schalteinrichtung der Gassteuerungsvorrichtung 10 wird durch die zeitgesteuerte Schaltung des Beatmungsgerätes zwischen Inspiration und Exspiration, getriggert. In der Regel sperrt die Schalteinrichtung den zweiten Gaskanal 12, sobald das Beatmungsgerät auf Exspiration umschaltet. Wahlweise kann die Schaltung zeitverzögert erfolgen. In der Regel gibt die Schalteinrichtung den zweiten Gaskanal 12 frei, wenn das Beatmungsgerät auf Inspiration schaltet.

In der Figur 1b ist ferner der Umgehungskanal 21 dargestellt. Der Umgehungskanal 21 erstreckt sich von der Öffnung und ermöglicht die Vorbeiführung des aus der Öffnung ausströmenden Gasstroms an dem Rückschlagventil 19 vorbei. Der Umgehungskanal 21 ist umlaufend um die Öffnung ausgebildet und verengt sich in seinem weiteren Verlauf zu einem Kanal, der sich in einer Richtung, parallel zu dem ersten Gaskanal erstreckt. Der Umgehungskanal 21 kann anschließend erneut in den ersten Gaskanal einmünden oder als separater Zweig den an dem Rückschlagventil vorbeigeführten Gasstrom abführen.

Der Umgehungskanal weist im Bereich der - nicht gezeigten - Öffnung einen Rand auf, auf den die Dichtung durch die Verschlusskappe 16 abgedichtet werden kann. Der Rand des Umgehungskanals 21 kann eine Hervorhebung aufweisen, hinter die an eine Kante der Dichtung eingreifen kann. Der Rand des Umgehungskanals 21 verhindert ein Verrutschen der Dichtung.

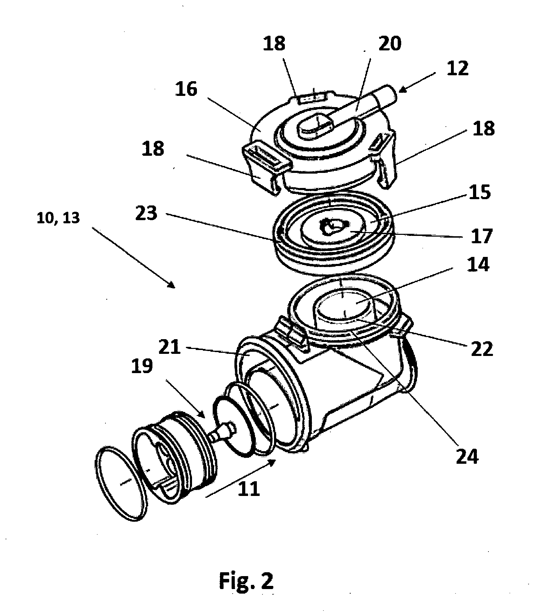

Figur 2 zeigt eine schematische Explosionsansicht der in Figur 1 gezeigten Gassteuerungsvorrichtung 10. Dabei sind der erste und der zweite Gaskanal 11, 12, das Rückschlagventil 19 sowie die Öffnung 14 mit der Dichtung 15 gezeigt.

Dargestellt ist auch die Verschlusskappe 16 mit dem Anschluss 20. Gezeigt ist die mittige Anordnung des Anschlusses 20, wobei ein Ende des Anschlusses 20 sich nach außen erstreckt und mit dem zweiten Gaskanal 12 verbindbar ist.

In der Figur 2 ist gezeigt, dass die Öffnung 14 einen Rand 22 aufweist. Der Rand 22 ist als Erhebung ausgebildet und dient als Ablage für das in die Dichtung 15 integriert ausgebildete Gewicht 17.

Die Dichtung 15 ist aus einem elastomeren Material, vorzugsweise einem Silikon mit einer Härte zwischen 15 bis 25 Shore A, insbesondere zwischen 18 und 22 Shore A ausgebildet. Die Dichtung 15 ist kreisförmig ausgebildet und weist eine Begrenzung 23 auf.

Die Begrenzung 23 kann als eine verstärkte Struktur oder als funktionale Hervorhebung ausgebildet sein. Die Begrenzung 23 ist eingerichtet, auf einem Rand 24 des Umgehungskanals 21 aufzuliegen. Die Dichtung 15 kann zwischen der Begrenzung 23 der Dichtung 15 und dem Gewicht 17 eine dünnere Materialstärke aufweisen als im Bereich des Randes 24 des Umgehungskanals 21 und des Bereichs der Dichtung 15, in dem das Gewicht 17, zumeist integral, angeordnet ist.

In der vorliegenden Ausführungsform weist die Dichtung 15 zwischen dem Rand 24 des Umgehungskanals 21 und dem Gewicht 17 eine gewölbte Struktur auf, die zusätzlich einer Verschiebung der Dichtung 15 auf der Öffnung 14 vorbeugen kann. In weiteren Ausführungsformen kann die Dichtung 15 zwischen dem Rand 24 des Umgehungskanals 21 und dem Gewicht 17 eine andere Struktur aufweisen oder strukturfrei ausgebildet sein.

Das Gewicht 17 der Dichtung 15 ist in Form einer Unterlegscheibe ausgebildet. Das Gewicht 17 ist aus einem Metall gebildet und in die Dichtung 17 integral ausgebildet. Das Gewicht 17 stabilisiert die Dichtung 15 auf der Öffnung 14.

Beim Aufsetzen der Verschlusskappe 16 auf die Öffnung 14 wird die Dichtung 15 im Bereich der Begrenzung 23 der Dichtung 15 an den Rand des Umgehungskanals 21 angedrückt und gehalten. Die Verschlusskappe 16 dichtet die Dichtung 15 nach außen ab und hält die Dichtung 15 in ihrer Position innerhalb der Verschlusskappe 16. Die Dichtung 15 wird somit innerhalb der Gassteuerungsvorrichtung 10 durch die aufgeklippte Verschlusskappe 16 auf dem Rand 24 des Umgehungskanals 21 angedrückt/gehalten. Die Dichtung 15 wird über die umlaufend ausgebildete, verstärkte Begrenzung 23 zwischen dem Rand 24 des Umgehungskanals 21 und der Verschlusskappe 16 eingeklemmt/abgedichtet.

Bereiche der Dichtung 15, die sich von der Begrenzung 23 unterscheiden, sind berührungsfrei zu der Verschlusskappe 16 ausgebildet. Diese Bereiche, können auch als Beaufschlagungsbereiche bezeichnet werden, da bei einer Freigabe des zweiten Gaskanals 12 durch die Schalteinrichtung diese Bereiche der Dichtung 15 mit einem Gasstrom/Druck beaufschlagt werden.

Die Dichtung 15 wird in einem Ruhezustand (ohne Gasströme) durch das Gewicht und ihre Struktur auf der Öffnung 14 gehalten. Ist die Schalteinrichtung eingerichtet, den zweiten Gaskanal 12 freizugeben, werden zusätzlich die Beaufschlagungsgbereiche der Dichtung 15 mit einem Gasstrom / Druck beaufschlagt, wodurch die Dichtung 15 auf die Öffnung 14 abgedichtet wird.

Ist die Schalteinrichtung eingerichtet, den zweiten Gaskanal 12 zu sperren, werden die Beaufschlagungsbereiche der Dichtung 15 nicht mit einem Gasstrom beaufschlagt, sodass der Gasstrom des ersten Gaskanals 11 ausreichend ist, um die Dichtung im Bereich der Beaufschlagungsbereiche anzuheben und den Gasstrom in den Umgehungskanal 21 zu führen.

Die in der Figur 2 gezeigte Verschlusskappe 16 umfasst Fortsätze 18, die Widerhaken ausbilden. Die Widerhaken greifen beim Aufklippen der Verschlusskappe 16 in Ausnehmungen ein, die an der Außenseite der Gassteuerungsvorrichtung 10 ausgebildet sind. In weiteren Ausführungsformen sind weitere Verschlussmöglichkeiten, beispielsweise eine Verrastung in Form eines Bajonettverschlusses denkbar. Ferner sind in der Figur 2 das Rückschlagventil 19 sowie der Umgehungskanal 21 dargestellt.

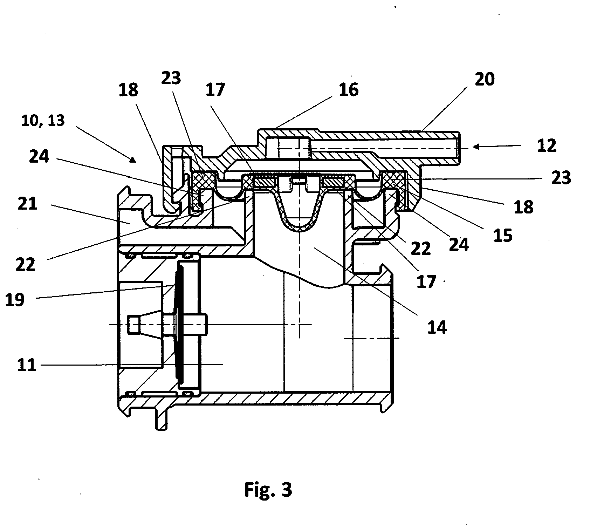

Figur 3 zeigt einen Längsschnitt durch die in den Figuren 1 und 2 gezeigte. Gassteuerungsvorrichtung 10. Dargestellt ist der erste Gaskanal 11 mit dem Rückschlagventil 19, der Öffnung 14 und der Dichtung 15 sowie der zweite Gaskanal 12. Zudem sind die Verschlusskappe 16 mit dem Anschluss 20 und der Umgehungskanal 21 dargestellt.

Über den ersten Gaskanal 11 kann ein inspiratorischer Gasstrom zu dem Patienten gefördert werden. Das Rückschlagventil 19 verhindert ein Rückströmen von Ausatemgas in das Beatmungsgerät über den ersten Gaskanal 11. Bei einer Blockade/Störung des exspiratorischen Zweiges des Beatmungsgerätes kann somit über den ersten Gaskanal 11 keine Exspiration des Gasstroms/Ausatemgases erfolgen.

Gezeigt ist zudem der Umgehungskanal 21, der sich von der Öffnung 14 aus erstreckt. Der Umgehungskanal 21 ist als umlaufender Kanal um die Öffnung 14 ausgebildet und erstreckt sich in seinem weiteren Verlauf zu einem Kanal, der sich in einer Richtung, parallel zu dem ersten Gaskanal 11 erstreckt. Durch die umlaufende Anordnung des Umgehungskanals kann eine große Menge an Ausatmengas gleichzeitig um das Rückschlagventil 19 vorbei und über die Öffnung 14 abgeführt werden. Der Umgehungskanal 21 kann in den inspiratorischen Zweig einmünden oder als separater exspiratorischer Zweig ausgebildet sein.

Die Dichtung 15 ist kreisförmig ausgebildet und weist in ihrem Zentrum ein Gewicht 17 auf. Das Gewicht 17 ist ringförmig/unterlegscheibenförmig ausgebildet. Das Gewicht 17 weist in seiner formbedingten Aussparung eine kegelförmige Ausgestaltung der Dichtung 15 auf, die sich in Richtung des ersten Gaskanals 11 erstreckt. Die kegelförmige Ausgestaltung bietet den Vorteil, dass die Dichtung 15 in ihrer Position auf der Öffnung 14 gegen verrutschen gesichert ist. In weiteren Ausführungsformen kann die Ausgestaltung der Dichtung 15 eine andere geometrische Form aufweisen, die geeignet ist, die Dichtung 15 in ihrer Position zu halten. Das Gewicht 17 ist in die Dichtung 15 integriert ausgebildet. Die Dichtung 15 weist im Bereich des Gewichts 17 eine größere Materialstärke auf. Dies bietet zum einen den Vorteil, dass das Gewicht 17 in die Dichtung 15 integrierbar ist und zum anderen durch die höhere Materialstärke ein zusätzliches Gewicht 17 erzeugt wird, dass die Dichtung 15 in ihrer Position hält. Das Gewicht 17 kann auch auf die Dichtung 15 aufgebracht ausgebildet sein.

Die in der Figur 3 gezeigte Verschlusskappe 16 ist auf der Öffnung 14 angeordnet, wobei die Verschlusskappe 16 im Bereich der Begrenzung 23 die Dichtung 15 auf den Rand des Umgehungskanals 21 andrückt, während die Dichtung 15 in den Beaufschlagungsbereichen berührungsfrei zu der Verschlusskappe 16 angeordnet ist.

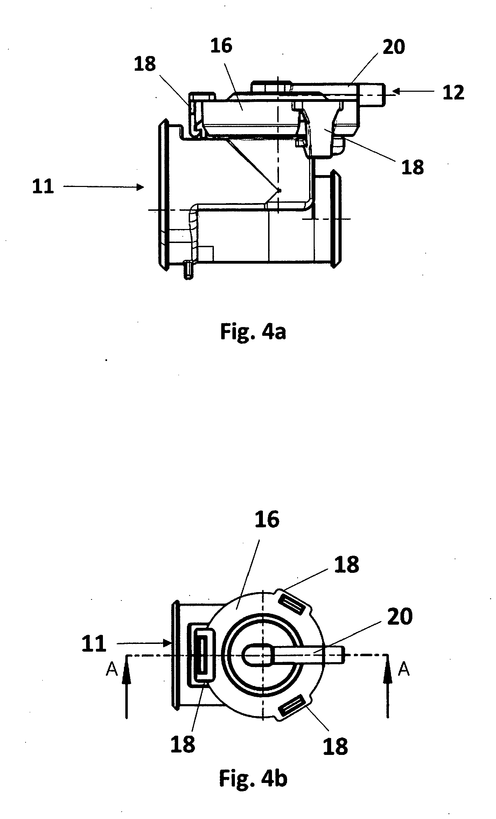

Figur 4a zeigt eine Seitenansicht der in den Figuren 1 bis 3 gezeigten Gassteuerungsvorrichtung 10. Dargestellt ist der erste Gaskanal 11 sowie die Verschlusskappe 16 mit den Fortsätzen 18 und dem Anschluss 20 für den - nicht gezeigten - zweiten Gaskanal.

In der Figur 4a ist zu erkennen, dass die Eingangsseite des ersten Gaskanals 11 größer dimensioniert ist als die Ausgangsseite. Die Eingangsseite umfasst neben dem ersten Gaskanal 11 auch den zu einem Kanal verengten Umgehungskanal 21. Somit umfasst die Eingangsseite des ersten Gaskanals 11 sowohl den in Inspirationsflussrichtung ausgerichteten ersten Gaskanal 11 sowie den entgegen der Inspirationsflussrichtung ausgerichteten Umgehungskanal 21.

Figur 4b zeigt eine Draufsicht auf die erfindungsgemäße Verschlusskappe 16 der in den Figuren 1 bis 4b gezeigten erfindungsgemäßen Gassteuerungsvorrichtung 10. Die Verschlusskappe 16 ist auf die - nicht gezeigte - Öffnung des ersten Gaskanals aufklippbar. Beim Aufklippen der Verschlusskappe 16 wird die Dichtung im Bereich des - nicht gezeigten - Gewichts von der Verschlusskappe 16 auf eine - in Figur 3 gezeigte - Erhebung 22 der Öffnung gedrückt und abgedichtet.

Die Verschlusskappe 16 weist einen Anschluss 20 für den - nicht gezeigten - zweiten Gaskanal 12 auf. Über den Anschluss 20 kann der Gasstrom des - nicht gezeigten - zweiten Gaskanals 12 auf die - nicht gezeigte - Dichtung aufgebracht werden, um die - nicht gezeigte - Öffnung zu verschließen. Die Verschlusskappe 16 umfasst ferner die Fortsätze 18 die zumeist Widerhaken ausbilden und eingerichtet sind in Ausnehmungen im Bereich der Öffnung einzugreifen. In weiteren Ausführungsformen kann die Verschlusskappe 16 weitere Fortsätze 18 aufweisen oder über ähnliche Verrastungselemente auf der Öffnung befestigbar sein.

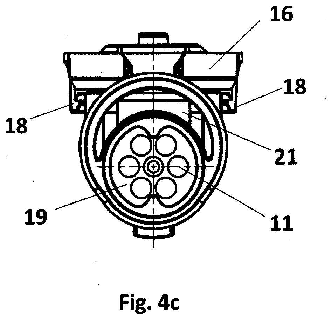

Figur 4c zeigt eine Draufsicht auf das Rückschlagventil 19 der in den Figuren 1 bis 4b gezeigten erfindungsgemäßen Gassteuerungsvorrichtung 10. Dabei wird aus Richtung des einströmenden Gasstroms, in Inspirationsflussrichtung, auf das Rückschlagventil 19 geblickt. Gezeigt ist auch der Umgehungskanal 21, über welchen bei einer Blockade/Störung des exspiratorischen Zweiges des Beatmungsgerätes der Gasstrom um das Rückschlagventil 19 herumführbar ist.

Dargestellt ist auch die Verschlusskappe 16 in Seitenansicht mit den Fortsätzen 18, mittels derer die Verschlusskappe 16 auf die - nicht gezeigte - Öffnung aufklippbar ist.

**Bezugszeichenliste**

| 1 | Beatmungsgerät |
|---|---|
| 10 | Gassteuerungsvorrichtung |
| 11 | erster Gaskanal |
| 12 | zweiter Gaskanal |
| 13 | Schalteinrichtung / Ventilblock |
| 14 | Öffnung |
| 15 | Dichtung |
| 16 | Verschlusskappe der Öffnung |
| 17 | Gewicht |
| 18 | Fortsätze der Verschlusskappe |
| 19 | Rückschlagventil |
| 20 | Anschluss |
| 21 | Umgehungskanal |
| 22 | Rand der Öffnung/ Erhebung |
| 23 | Begrenzung der Dichtung |
| 24 | Rand des Umgehungskanals |
| 25 | Gasstrom erste Richtung |
| 26 | Gasstrom zweite Richtung |
| 27 | Beatmung-Schlauch |
| 28 | Schlauchstutzen |
| 29 | Umgebung |
| 30 | Gasquelle, Gebläse |

**Patentansprüche**

1. Gassteuerungsvorrichtung (10) für ein Beatmungsgerät (1) mit Atemgasquelle (30) und Beatmungsschlauch (27) umfassend einen ersten Gaskanal (11) mit einem ersten Ventil (19), wobei der erste Gaskanal (11) und das Ventil (19) eingerichtet sind, einen unter Druck stehenden Gasstrom in einer ersten Richtung (25) passieren zu lassen und einen unter Druck stehenden Gasstrom in einer zweiten Richtung (26) zu sperren, wobei der Gasstrom der zweiten Richtung (26) Atemgas der Exspiration ist und der Gasstrom der ersten Richtung (25) Atemgas der Inspiration ist, wobei eine Öffnung (14) von dem ersten Gaskanal (11) abzweigt und zu einem Umgehungskanal (21), für das Ventil (19), führt, wobei ein zweites Ventil (15, 16) eingerichtet ist einen Gasstrom vom dem ersten Gaskanal (11) zu dem Umgehungskanal (21) zu sperren und/oder zumindest zeitweise zu ermöglichen, wobei eine Schalteinrichtung (13) eingerichtet ist das zweite Ventil (15, 16) zu steuern, einen Gasstrom vom ersten Gaskanal (11) zu dem Umgehungskanal (21) zu sperren und/oder zumindest zeitweise zu ermöglichen, und dass das Ventil (15, 16) als pneumatisches Ventil ausgeführt ist, wobei eine Dichtung (15) des Ventils die gasleitende Verbindung zwischen Öffnung (14) und Umgehungskanal (21) verschließt oder öffnet und wobei ein zweiter Gaskanal (12) eingerichtet ist einen pneumatischen Steuerdruck auf die Oberfläche der Dichtung zu leiten, und dass der zweite Gaskanal (12) mit dem Beatmungsschlauch (27) oder dem Gaskanal (11) oder der Atemgasquelle (30) pneumatisch verbunden ist und die Schalteinrichtung (13) eingerichtet ist die pneumatische Verbindung zumindest zeitweise zu öffnen oder zu verschließen,

**dadurch gekennzeichnet, dass** die Schalteinrichtung (13) dazu eingerichtet ist bei einer Blockade oder Störung eines exspiratorischen Zweiges des Beatmungsgerätes, die ein Ausatmen des Patienten verhindert oder erschwert, den zweiten Gaskanal (12) zu sperren, sodass durch den gesperrten Gaskanal (12) der Gasstrom des zweiten Gaskanals (12), der auf die Dichtung (15) wirkt, zum Erliegen kommt und wodurch der Gasstrom des ersten Gaskanals (11) die Dichtung, die auf der Öffnung aufliegt, anheben kann, sodass der Gasstrom des ersten Gaskanals über einen Umgehungskanal (21) um das erste Ventil (19) herum in den ersten Gaskanal (11) und von dort zumindest teilweise in die Umgebung geführt wird.

2. Gassteuerungsvorrichtung (10) nach Anspruch 1, **dadurch gekennzeichnet, dass** das erste Ventil ein pneumatisch betätigtes Rückschlagventil (19) ist, welches durch die Kraft des unter Druck stehenden Gasstroms in der zweiten Richtung (26) betätigt wird und somit der Gasstrom das Rückschlagventil (19) in einer zweiten Richtung (26) nicht passieren kann.

3. Gassteuerungsvorrichtung (10) nach einem der voranstehenden Ansprüche, **dadurch gekennzeichnet, dass** die Atemgasquelle (30) Atemgas aus der Umgebung (29) ansaugt und entlang dem ersten Gaskanal (11) für die Inspiration in der ersten Richtung (25) zu einem Schlauchstutzen (28) und dem Beatmungsschlauch (27) befördert, wobei der Gasstrom das geöffnete Rückschlagventil (19) passiert.

4. Gassteuerungsvorrichtung (10) nach einem der voranstehenden Ansprüche, **dadurch gekennzeichnet, dass** die mit dem Steuerdruck beaufschlagte Dichtung (15) eingerichtet ist, sich dichtend an den Rand (22) der Öffnung (14) anzulegen und so einen Gasstrom von der Öffnung in den Umgehungskanal (21) zu verhindern.

5. Gassteuerungsvorrichtung (10) nach einem der voranstehenden Ansprüche, **dadurch gekennzeichnet, dass** der erste Gaskanal (11) die Öffnung (14) zum Ausströmen von Gas mit der Dichtung (15) und einer Verschlusskappe (16) aufweist.

6. Gassteuerungsvorrichtung (10) nach einem der voranstehenden Ansprüche, **dadurch gekennzeichnet, dass** die Dichtung (15) auf der Öffnung (14) aufliegend angeordnet ist und ein Gewicht (17) umfasst, wobei das Gewicht (17) ringförmig in der Mitte der Dichtung (15) ausgebildet ist und wobei die Dichtung (15) aus einem elastomeren Material ausgebildet ist und das Gewicht (17) aus einem Metall ausgebildet ist.

7. Gassteuerungsvorrichtung (10) nach einem der voranstehenden Ansprüche, **dadurch gekennzeichnet, dass** die Dichtung (15) eine Ausprägung aufweist, die im Bereich des Gewichts (17) in Richtung des ersten Gaskanals (11) weisend ausgebildet ist.

8. Gassteuerungsvorrichtung (10) nach Anspruch 5, **dadurch gekennzeichnet, dass** die Verschlusskappe (16) eingerichtet ist, auf die Öffnung (14) aufsetzbar zu sein und wobei die Verschlusskappe (16) einen Anschluss (20) umfasst, der mit dem zweiten Gaskanal (12) verbindbar ist.

9. Gassteuerungsvorrichtung (10) nach Anspruch 8, **dadurch gekennzeichnet, dass** die Schalteinrichtung (13) eingerichtet ist, bei einem Normalbetrieb des exspiratorischen Zweiges den zweiten Gaskanal (12) freizugeben und einen Gasstrom/ Druck über den zweiten Gaskanal (12) und den Anschluss (20) auf die Dichtung (15) zu leiten und die Öffnung (14) zu schließen.

10. Gassteuerungsvorrichtung (10) nach Anspruch 6, **dadurch gekennzeichnet, dass** das Ventil (15, 16) eingerichtet ist, bei einem gesperrten zweiten Gaskanal (12) einem Gasstrom des ersten Gaskanals (11) einen Durchlass durch die Öffnung (14) zu ermöglichen.

11. Beatmungsgerät umfassend eine Gassteuerungsvorrichtung (10) gemäß einem der vorhergehenden Ansprüche.

**Claims**

1. A gas control device (10) for a ventilator (1) with a breathing gas source (30) and a ventilation tube (27) comprising a first gas channel (11) with a first valve (19), wherein the first gas channel (11) and the valve (19) are configured to let a pressurized gas flow pass in a first direction (25) and to block a pressurized gas flow in a second direction (26), wherein the gas flow in the second direction (26) is exhalation breathing gas and the gas flow in the first direction (25) is inhalation breathing gas, wherein an opening (14) branches off from the first gas channel (11) and leads to a bypass channel (21) for the valve (19), wherein a second valve (15, 16) is configured to block and/or at least at times enable a gas flow from the first gas channel (11) to the bypass channel (21), wherein a switching apparatus (13) is configured to control the second valve (15, 16) and to block and/or at least at times enable a gas flow from the first gas channel (11) to the bypass channel (21), and that the valve (15, 16) is designed as a pneumatic valve, wherein a seal (15) of the valve closes or opens the gas-conducting connection between the opening (14) and the bypass channel (21) and wherein a second gas channel (12) is configured to conduct a pneumatic control pressure onto the surface of the seal (15), and that the second gas channel (12) is pneumatically connected to the ventilation tube (27) or the gas channel (11) or the breathing gas source (30) and the switching apparatus (13) is configured to at least at times open or close the pneumatic connection, **characterized in that** the switching apparatus (13) is configured such that, if an exhalation branch of the ventilator is blocked or disrupted in a way that prevents or impedes an exhalation of the patient, the second gas channel (12) is blocked so that the blocked gas channel (12) causes the gas flow in the second gas channel (12), which acts on the seal (15), to come to a stop and as a result of which the gas flow in the first gas channel (11) can lift the seal, which lies on the opening, so that the gas flow in the first gas channel is guided via a bypass channel (21) around the first valve (19) into the first gas channel (11) and from there at least partially into the environment.

2. The gas control device (10) according to claim 1, **characterized in that** the first valve is a pneumatically actuated check valve (19), which is actuated by the force of the pressurized gas flow in the second direction (26) and thus the gas flow cannot pass the check valve (19) in a second direction (26).

3. The gas control device (10) according to one of the preceding claims, **characterized in that** the breathing gas source (30) draws in breathing gas from the environment (29) and conveys it along the first gas channel (11) for inhalation in the first direction (25) to a tube connector (28) and the ventilation tube (27), wherein the gas flow passes the opened check valve (19).

4. The gas control device (10) according to one of the preceding claims, **characterized in that** the seal (15) to which the control pressure is applied is configured to lay in a sealing manner against the edge (22) of the opening (14) and thus prevent gas from flowing from the opening into the bypass channel (21).

5. The gas control device (10) according to one of the preceding claims, **characterized in that** the first gas channel (11) has the opening (14) for the outflow of gas with the seal (15) and a cap (16).

6. The gas control device (10) according to one of the preceding claims, **characterized in that** the seal (15) is arranged lying against the opening (14) and comprises a weight (17), wherein the weight (17) is formed annularly in the center of the seal (15) and wherein the seal (15) is formed from an elastomer material and the weight (17) is formed from a metal.

7. The gas control device (10) according to one of the preceding claims, **characterized in that** the seal (15) has a shape that is designed in the region of the weight (17) to face in the direction of the first gas channel (11).

8. The gas control device (10) according to claim 5, **characterized in that** the cap (16) is configured to be placeable onto the opening (14) and wherein the cap (16) has a connection (20) that can be connected to the second gas channel (12).

9. The gas control device (10) according to claim 8, **characterized in that** the switching apparatus (13) is configured to release the second gas channel (12) during normal operation of the exhalation branch and to conduct a gas flow/pressure onto the seal (15) via the second gas channel (12) and the connection (20) and to close the opening (14).

10. The gas control device (10) according to claim 6, **characterized in that** the valve (15, 16) is configured to enable a gas flow in the first gas channel (11) to pass through the opening (14) when the second gas channel (12) is blocked.

11. A ventilator comprising a gas control device (10) according to one of the previous claims.

## Revendications

1. Dispositif de commande de gaz (10) pour un appareil respiratoire (1) avec source de gaz respiratoire (30) et tube respiratoire (27) comprenant un premier canal de gaz (11) avec une première soupape (19), le premier canal de gaz (11) et la soupape (19) étant agencés pour laisser passer un flux de gaz sous pression dans une première direction (25) et pour bloquer un flux de gaz sous pression dans une deuxième direction (26), le flux de gaz de la deuxième direction (26) étant le gaz respiratoire de l'expiration et le flux de gaz dans la première direction (25) étant le gaz respiratoire de l'inspiration, une ouverture (14) bifurquant du premier canal de gaz (11) et conduisant à un canal de dérivation (21), pour la soupape (19), une deuxième soupape (15, 16) étant agencée pour bloquer et/ou permettre au moins temporairement un flux de gaz du premier canal de gaz (11) vers le canal de dérivation (21), un dispositif de commutation (13) étant agencé pour commander la deuxième soupape (15, 16), pour bloquer et/ou permettre au moins temporairement un flux de gaz du premier canal de gaz (11) vers le canal de dérivation (21), et en ce que la soupape (15, 16) est réalisée comme une soupape pneumatique, un joint (15) de la soupape fermant ou ouvrant le raccordement conducteur de gaz entre l'ouverture (14) et le canal de dérivation (21), et un deuxième canal de gaz (12) étant agencé pour diriger une pression de commande pneumatique sur la surface du joint (15), et en ce que le deuxième canal de gaz (12) est raccordé de façon pneumatique avec le tube respiratoire (27) ou le canal de gaz (11) ou la source de gaz respiratoire (30) et le dispositif de commutation (13) étant agencé pour ouvrir ou fermer au moins temporairement le raccordement pneumatique, **caractérisé en ce que** le dispositif de commutation (13) est agencé pour fermer le deuxième canal de gaz (12) lors d'un blocage ou d'une défaillance d'une branche expiratoire de l'appareil respiratoire qui empêche ou entrave une expiration du patient, de telle sorte que, par le canal de gaz (12) fermé, le flux de gaz du deuxième canal de gaz (12) agissant sur le joint (15) s'arrête, ce par quoi le flux de gaz du premier canal de gaz (11) peut soulever le joint reposant sur l'ouverture, de telle sorte que le flux de gaz du premier canal de gaz est conduit par un canal de dérivation (21) dans le premier canal de gaz (11) autour de la première soupape (19) et étant conduit au moins partiellement vers l'environnement à partir de celui-ci.

2. Dispositif de commande de gaz (10) selon la revendication 1, **caractérisé en ce que** la première soupape est un clapet antiretour (19) actionné de façon pneumatique, lequel est actionné par la force du flux de gaz sous pression dans la deuxième direction (26) et le flux de gaz ne pouvant ainsi pas passer le clapet antiretour (19) dans une deuxième direction (26).

3. Dispositif de commande de gaz (10) selon l'une des revendications précédentes, **caractérisé en ce que** la source de gaz respiratoire (30) aspire du gaz respiratoire de l'environnement (29) et le transporte le long du premier canal de gaz (11) pour l'inspiration dans la première direction (25) à un embout de tube (28) et au tube respiratoire (27), le flux de gaz passant le clapet antiretour (19) ouvert.

4. Dispositif de commande de gaz (10) selon l'une des revendications précédentes, **caractérisé en ce que** le joint (15) auquel la pression de commande est appliquée est agencé pour s'appliquer de façon étanche au bord (22) de l'ouverture (14) et pour empêcher ainsi un flux de gaz de l'ouverture vers le canal de dérivation (21).

5. Dispositif de commande de gaz (10) selon l'une des revendications précédentes, **caractérisé en ce que** le premier canal de gaz (11) présente l'ouverture (14) pour l'écoulement du gaz avec le joint (15) et un bouchon de fermeture (16).

6. Dispositif de commande de gaz (10) selon l'une des revendications précédentes, **caractérisé en ce que** le joint (15) est disposé en appui sur l'ouverture (14) et comprend un poids (17), le poids (17) étant conçu de façon annulaire au milieu du joint (15) et le joint (15) étant composé d'un matériau élastomère et le poids (17) étant composé d'un métal.

7. Dispositif de commande de gaz (10) selon l'une des revendications précédentes, **caractérisé en ce que** le joint (15) présente une empreinte qui est conçue avec orientation dans la direction du premier canal de gaz (11) dans la zone du poids (17).

8. Dispositif de commande de gaz (10) selon la revendication 5, **caractérisé en ce que** le bouchon de fermeture (16) est agencé pour pouvoir être placé sur l'ouverture (14) et le bouchon de fermeture (16) comprenant un raccord (20), lequel est raccordable avec le deuxième canal de gaz (12).

9. Dispositif de commande de gaz (10) selon la revendication 8, **caractérisé en ce que** le dispositif de commutation (13) est agencé pour ouvrir le deuxième canal de gaz (12) pendant un fonctionnement normal de la branche expiratoire et pour conduire un flux de gaz/une pression par le deuxième canal de gaz (12) et le raccord (20) au joint (15) et pour fermer l'ouverture (14).

10. Dispositif de commande de gaz (10) selon la revendication 6, **caractérisé en ce que** la soupape (15, 16) est agencée pour permettre à un flux de gaz du premier canal de gaz (11) un passage par l'ouverture (14), lorsqu'un deuxième canal de gaz (12) est fermé.

11. Appareil respiratoire comprenant un dispositif de commande de gaz (10) selon l'une des revendications précédentes.

**Fig. 1a**

Fig. 1b

**Fig. 2**

**Fig. 3**

**Fig. 4a**

**Fig. 4b**

**Fig. 4c**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017059667 A
- DE 202017005964